# EUROPEAN PATENT APPLICATION

(11) **EP 2 366 795 A1**
(43) Date of publication of application: **21.09.2011**
(21) Application number: 11157150.1
(22) Date of filing: 07.03.2011
(51) Int. Cl.: C12Q 1/04, C12M 1/26

(54) **Storing method of measuring apparatus of airborne floating bacteria**

(30) Priority: 17.03.2010 JP 2010061268
(71) Applicant: Hitachi Plant Technologies, Ltd., Tokyo (JP)
(72) Inventor: Yamamoto, Harumasa, Tokyo (JP)
(74) Representative: Beetz & Partner

(57) **Abstract**

[Problem] To present a storing method of a measuring apparatus of airborne floating bacteria capable of preventing contamination of the measuring apparatus by airborne floating bacteria and growth of airborne floating bacteria in the measuring apparatus during storage of the measuring apparatus of airborne floating bacteria.

[Solving Means] In a storing method of a measuring apparatus of airborne floating bacteria for capturing airborne floating bacteria on a gel of a capturing carrier, forming into a sol in the measuring apparatus, and measuring the light emission of the ATP, the aseptic water existing in the piping system L of the measuring apparatus is displaced with alcohol, and dried, and the measuring apparatus of airborne floating bacteria is stored.

## Description

### [Technical Field]

The present invention relates to a storing method of a measuring apparatus of airborne floating bacteria, and more particularly to a storing method of a measuring apparatus of airborne floating bacteria for preventing contamination of the inside of the measuring apparatus of airborne floating bacteria during shut-down period of the measuring apparatus of airborne floating bacteria.

### [Background Art]

The present applicant formerly proposed a method of counting the number of cells in airborne floating bacteria in a manufacturing environment of aseptic pharmaceutical preparations or a manufacturing environment of food products, in which the quantity of adenosine triphosphate (ATP) derived from viable cells in the airborne floating bacteria is determined by measuring the light emission quantity due to a biological light emission reaction, and the number of cells in the airborne floating bacteria is counted on the basis of the obtained result (see, for example, patent literature 1).

In this method, airborne floating bacteria are captured on a carrier containing gelatin by using a capturing machine, and the carrier containing gelatin is heated in the measuring apparatus, and is diluted in an aseptic water to transfer the phase from gel to sol, and the ATP is extracted from the captured bacteria, and the light emission is measured, and this measuring apparatus operating on the ATP method requires a piping system filled with two kinds of aseptic water, that is, a warm water system for supplying aseptic water for diluting the carrier, and a dispensing and dividing system of a syringe pump for dispensing and dividing samples and reagents.

If the place of installation of the measuring apparatus is an aseptic space, contamination by bacteria does not occur, but if the measuring apparatus is installed in a non-aseptic environment, the leading end of the piping, the leading end of the warm water dispensing nozzle, and the leading end of the sampling and dispensing nozzle are exposed to air carrying bacteria, and the piping system filled with aseptic water may be contaminated from the opening part of the leading end, and there is a risk that the contamination may spread over widely inside of the piping.
In the warm water system, since the warm water heater for super-heating the warm water provided at a later stage of the pump for pumping out the warm water cannot be sterilized easily, and if the piping of the warm water system is contaminated by bacteria, a filter is provided to capture the bacteria by a hollow fiber filter so that the bacteria may not invade at the time of dilution of the capturing carrier, but there is a risk of growth of captured bacteria in the filter.

Accordingly, when shutting down the measuring apparatus for a short period, the apparatus is stopped in the state of the inside of the piping filled with aseptic water, and the nozzles and other parts that can be sterilized upon re-start of operation are sterilized in an autoclave, the piping of the sampling and dispensing system is stored together with ATP extraction reagents or ATP eliminating reagents, and the ATP contained in bacteria is consumed in the cleaning process, and an aseptic state is recovered.

Or when shutting down the measuring apparatus for longer period (for example, during summer or New Year's vacation for a week or about ten days, longer than a short period), the piping system is discharged of aseptic water by sending in air, and stored in this state, and when resuming the operation, the operation is re-started in the same manner as in the shut-down for a short period.

However, the aseptic water in the piping partly remains in the piping at positions different in inside diameter or height in the warm water heater, and the aseptic water cannot be discharged completely, and remaining water drops also scatter about on the surface of the piping, and the moisture is hardly evaporated but is left over, and bacteria may grow in such positions.
Accordingly when resuming the operation, the reagents may be washed out by cleaning with a huge volume of cleaning water, or if not cleaned completely, all parts of the piping system should be replaced, and a tremendous amount of process and cost may be required before re-starting the operation.

### [Prior art literature]

### [Patent literature]

[Patent literature 1] Japanese Patent Application Laid-Open No. 2009-139115

### [Summary of the Invention]

[Problems to be Solved by the Invention]

The present invention is intended to present a storing method of a measuring apparatus of airborne floating bacteria, capable of preventing contamination of the measuring apparatus by airborne floating bacteria, or growth of airborne floating bacteria in the measuring apparatus, during storage of the measuring apparatus of airborne floating bacteria.

### [Means for Solving the Problems]

To achieve the object, a first aspect of the invention relates to a storing method of a measuring apparatus of airborne floating bacteria, characterized by capturing airborne floating bacteria on a gel of a capturing carrier, forming into a sol in the measuring apparatus, and measuring the light emission of the ATP, in which the aseptic water existing in the piping system of the measuring apparatus is displaced with alcohol, and dried, and the measuring apparatus of airborne floating bacteria is stored.

In this case, it is possible to exchange a hollow fiber filter when resuming operation of the measuring apparatus of airborne floating bacteria.

To achieve the object, a second aspect of the invention relates to a storing method of a measuring apparatus of airborne floating bacteria, characterized by capturing airborne floating bacteria on a gel of a capturing carrier, forming into a sol in the measuring apparatus, and measuring the light emission of the ATP, in which the nozzle section of the piping system of the measuring apparatus is filled with alcohol, and the measuring apparatus of airborne floating bacteria is stored in this state.

In this case, it is possible to resume operation of the measuring apparatus of airborne floating bacteria, by evaporating the alcohol packed in the nozzle section of the piping system of the measuring apparatus at the time of autoclave sterilization.

### [Effects of the Invention]

According to the storing method of the measuring apparatus of airborne floating bacteria in the first aspect of the invention, in the storing method of the measuring apparatus of airborne floating bacteria for capturing airborne floating bacteria on a gel of a capturing carrier, forming into a sol in the measuring apparatus, and measuring the light emission of the ATP, the aseptic water existing in the piping system of the measuring apparatus is displaced with alcohol, and dried, and the measuring apparatus of airborne floating bacteria is stored, and therefore when storing the measuring apparatus for a long or intermediate period, it is capable of preventing contamination of the measuring apparatus by airborne floating bacteria during the shutdown period, or growth of airborne floating bacteria in the measuring apparatus, and it is possible to simplify or omit the cleaning process when resuming operation of the measuring apparatus, and the cost and labor of maintenance works of the measuring apparatus can be saved, and the precision of measurement of airborne floating bacteria can be enhanced.

By exchanging a hollow fiber filter not resistant to alcohol when resuming operation of the measuring apparatus of airborne floating bacteria, the performance of the measuring apparatus can be maintained.

According to the storing method of the measuring apparatus of airborne floating bacteria in the second aspect of the invention, in the storing method of the measuring apparatus of airborne floating bacteria for capturing airborne floating bacteria on a gel of a capturing carrier, forming into a sol in the measuring apparatus, and measuring the light emission of the ATP, in which the nozzle section of the piping system of the measuring apparatus is filled with alcohol, and the measuring apparatus of airborne floating bacteria is stored in this state, and therefore when storing the measuring apparatus for a short period, it is capable of preventing contamination of the measuring apparatus by airborne floating bacteria during the shutdown period, or growth of airborne floating bacteria in the measuring apparatus, and it is possible to simplify or omit the cleaning process when resuming operation of the measuring apparatus, and the cost and labor of maintenance works of the measuring apparatus can be saved, and the precision of measurement of airborne floating bacteria can be enhanced.

In this case, it is possible to resume operation of the measuring apparatus of airborne floating bacteria, by evaporating the alcohol packed in the nozzle section of the piping system of the measuring apparatus at the time of autoclave sterilization, so that the operation of the measuring apparatus may be resumed easily.

### [Brief Description of the Drawings]

Fig. 1 is an explanatory diagram showing an example of a measuring apparatus of airborne floating bacteria conforming to a storing method of a measuring apparatus of airborne floating bacteria of the present invention.
Fig. 2 is a flowchart of storage process for shutting down the measuring apparatus of airborne floating bacteria for a long or intermediate period.
Fig. 3 is a flowchart of process of replacing the aseptic water in the piping system with alcohol.
Fig. 4 is a flowchart of procedure for resuming operation of the measuring apparatus of airborne floating bacteria after a shutdown.
Fig. 5 is a flowchart of storage process for shutting down the measuring apparatus of airborne floating bacteria for a short period.
Fig. 6 shows the state of the nozzle when shutting down the measuring apparatus of airborne floating bacteria for a short period, in which (a) shows the piping system of warm water system, and (b) shows the piping of dispensing and dividing system.

### [Best Mode for Carrying Out the Invention]

A preferred embodiment of the storing method of a measuring apparatus of airborne floating bacteria according to the present invention is described below while referring to the accompanying drawings.

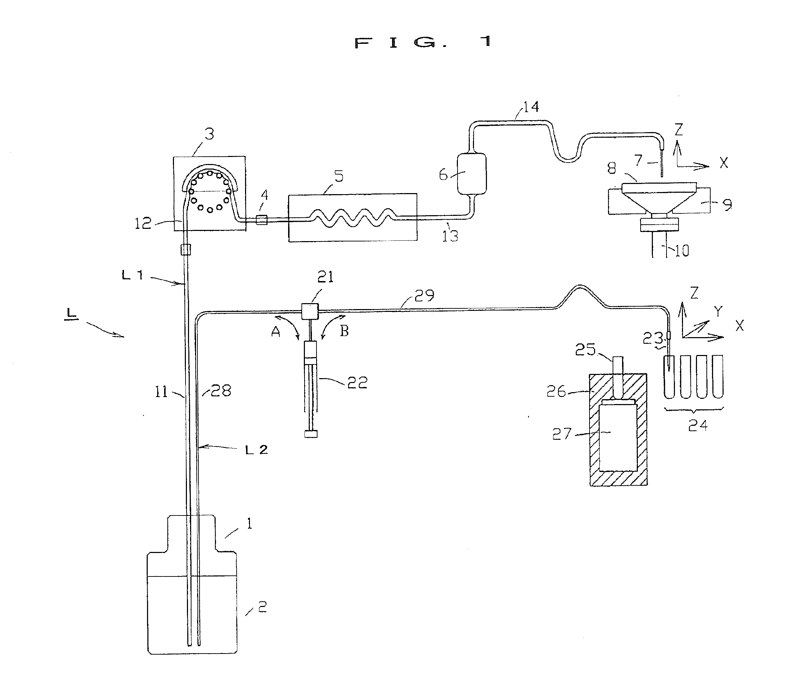

Fig. 1 shows an example of a measuring apparatus of airborne floating bacteria conforming to a storing method of a measuring apparatus of airborne floating bacteria of the present invention.

The measuring apparatus of airborne floating bacteria is used to determine the amount of ATP derived from the viable cells of the airborne floating bacteria by measuring the light emission quantity due to a biological light emission reaction, and to count the number of cells in the airborne floating bacteria on the basis of this finding, and is provided with a piping system L, more specifically a warm water system L1 as the piping system L handling aseptic water, and a dispensing and dividing system L2.

First, the warm water system L1 is composed of an aseptic water bottle 1 storing an aseptic water 2, a piping 11, a tube pump or other pump 3, a piping connector 4 for connecting between a peristaltic tube 12 of this pump 3 (when a tube pump is used as the pump 3) and a fixed piping 13, a warm water heater 5 for heating the aseptic water, a hollow fiber filter 6, a movable part piping 14, and a warm water nozzle 7.
The aseptic water bottle 1 is used commonly, in this embodiment, by the warm water system L 1 and the dispensing and dividing system L2, but it may be composed in different systems.
The warm water nozzle 7 is driven by a drive mechanism (not shown) so as to be movable in directions of two axes X and Y, and is designed to move between a discharge position (not shown) and the upper space of a capturing cartridge 8 containing a capturing carrier.
While the capturing cartridge 8 is being heated by a dissolving heater 9, and a sample diluted in warm water (aseptic water 2) is collected by way of a piping 10.

On the other hand, the dispensing and dividing system L2 is composed of the aseptic water bottle 1 (commonly used with the warm water system L1) storing the aseptic water 2, a syringe pump 22 designed to change over the path between direction A and direction B by means of a piping 28 and a three-way valve 21, a piping 29, and a dispensing and dividing nozzle 23.
When the aseptic water 2 is sucked into the syringe pump 22, the three-way valve 21 is changed over to direction A, and when the aseptic water 2 is sent out to the dispensing and dividing nozzle 23, or when the sample is sucked in from the dispensing and dividing nozzle 23, the three-way valve 21 is changed over to direction B.
The dispensing and dividing nozzle 23 is driven by a drive mechanism (not shown) so as to be movable in directions of three axes X, Y, and Z, and is designed to move the ATP elimination reagent, the ATP extraction reagent, the light emission reagent, the nozzle cleaning aseptic water 2, and others to a plurality of tubes 24 containing the reagents and a light emission measurement tube 25, so that the samples and the reagents can be dispensed and divided.
The light emission measurement tube 25 is a photomultiplier 27 installed in a darkroom 26, and the light emission reagent and the sample containing ATP are caused to react in the light emission measurement tube 25, and the magnitude of reaction is observed as the quantity of emission of light in the photomultiplier 27.

In the embodiment, the aseptic water bottle 1 storing the aseptic water 2 is used commonly by the warm water system L1 and the dispensing and dividing system L2, by immersing and disposing the piping 11 of the warm water system L 1 and the piping 28 of the dispensing and dividing system L2 in the aseptic water 2, but it may be also provided individually.
It is preferred to use fluorine-resin or silicone-rubber tubes for the piping 11, the peristaltic tube 12, the fixed piping 13, and the movable part piping 14 of the warm water system L1, and to use PEEK resin (polyether ether ketone resin) tubes for the pipings 28, 29 of the dispensing and dividing system L2 from the viewpoint of resistance to pressure of the syringe pump.
For the warm water nozzle 7 of the warm water system L1 and the dispensing and dividing nozzle 23 of the dispensing and dividing system L2, it is preferred to use stainless steel, or PEEK resin (polyether ether ketone resin).

### [Storing method when shutting down the measuring apparatus of airborne floating bacteria for a long or intermediate period]

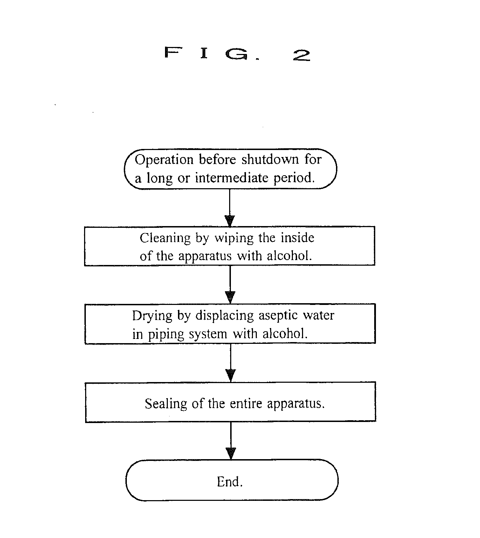

Fig. 2 shows an outline flow of a storing method when shutting down the measuring apparatus of airborne floating bacteria for a long or intermediate period.
The inside of the measuring apparatus of airborne floating bacteria may include positions contaminated with ATP or other bacteria due to contact with hands of an operator manipulating the measuring apparatus, or positions stained with reagents or scattering carriers including the diluted and dissolved gelatin, or the like, and the inside of the measuring apparatus must be cleaned by wiping contaminated positions and other parts of the apparatus with alcohol.
Any alcohol may be used, such as ethanol, isopropyl alcohol, or other general disinfecting alcohols. These alcohols, that is, ethanol, isopropyl alcohol, and mixture of ethanol and isopropyl alcohol are designated in the Japanese Pharmacopoeia as disinfectant compounds, and are easily available at low price. Other alcohols than mentioned above may be used as far as satisfying the requirements, such as ease of blending with water at an arbitrary rate, capability to kill bacteria, capability of delaying growth of bacteria not disinfected better than aseptic water, or resistance to chemicals of the material in the piping system L.
Next, the aseptic water in the piping system L is displaced with alcohol, and dried.
Finally, the entire measuring apparatus is packed and sealed with a nylon or other synthetic resin sheet.
It is also possible to compose so that only the piping system L may be taken out as an independent unit. In this case, only the piping system L may be taken out as a unit, and enclosed.

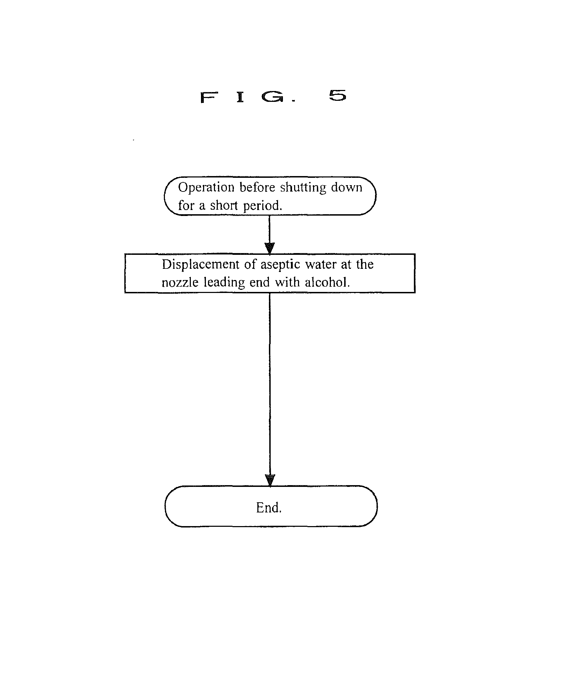

Fig. 5 shows a detail of a process of replacing the aseptic water in the piping system L with alcohol.
The aseptic water bottle 1 is taken out, and the leading ends of the piping 11 of the warm water system L 1 and the piping 28 of the dispensing and dividing system L2 are exposed.
Next, the pump 3 of the warm water system L 1 is put into operation, the water is discharged from the warm water nozzle 7, and the aseptic water in the piping of the warm water system L 1 is discharged.
On the other hand, the three-way valve 21 of the dispensing and dividing system L2 is changed over to direction A, and air is sucked into the syringe pump 22, and the three-way vale 21 is changed over to direction B, and the water is discharged from the dispensing and dividing nozzle 23, and the aseptic water is discharged from the piping of the dispensing and dividing system L2.
At the position of the aseptic water bottle 1, a bottle filled with alcohol is set in place of the aseptic water bottle 1.
The pump 3 of the warm water system L 1 is put into operation, and the alcohol is sucked in from the piping 11 of the warm water system L1, and the piping of the warm water system L 1 is filled with the alcohol, and the pump 3 is operated continuously until the alcohol in the piping is replaced two or three times.
As a result, the water staying inside the warm water heater 5, inside the hollow fiber filter 6, and inside the movable part piping 14 is capable of maintaining the alcohol at high concentration which is diluted locally if merely filled with alcohol, by operating continuously until the alcohol in the piping is replaced two or three times.
Similarly, the piping of the dispensing and dividing system L2 is filled with alcohol, and the pump is operated continuously until the alcohol in the piping is replaced two or three times.
Then, the alcohol bottle is detached, and by repeating the operation of the syringe pump 22 while operating the pump 3 continuously and changing over the three-way valve 21, air is sent into the pipings, and the alcohol staying inside the piping is evaporated.
The operation time is not particularly specified, but it is preferred to operate for about 30 to 60 minutes.
As the drying time becomes longer, the alcohol remaining inside can be evaporated in proportion to the operation time, but if bacteria are contained in the sucked air, a new contamination risk is involved, and operation for a long time is not preferred.
In the meantime, if the alcohol is not dried completely and is left over in the piping, only few species of bacteria can grow in the alcohol.
If the dead bacteria, or viable but non-culturable bacteria (VNC bacteria) are left over in the piping system L, by cleaning the piping when resuming the operation, an aseptic state may be recovered more easily than when shut down in a state filled with aseptic water.

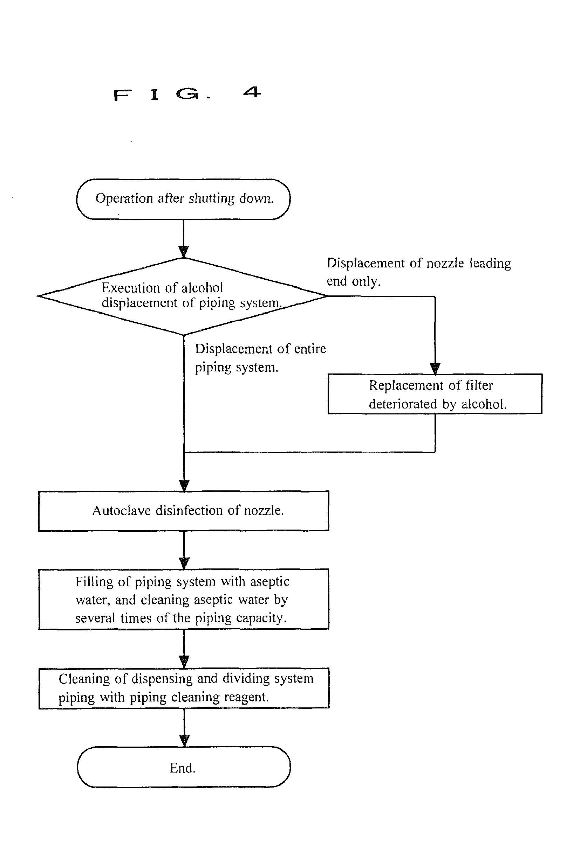

Fig. 4 shows an operation flow when resuming the operation of the measuring apparatus of airborne floating bacteria after shutting down for a long or intermediate period.
When shut down for a long or intermediate period, since the entire piping system L is displaced with alcohol, in particular, in the case of a rayon-made hollow fiber filter widely used as the hollow fiber filter 6, since the filter is not resistant to alcohol, the hollow fiber filter 6 is exchanged, and the warm water nozzle 7, the movable part piping 14, and the dispensing and dividing nozzle 23 are sterilized in autoclave.
Besides, other parts that can be easily detached such as the syringe pump 22 can be sterilized in autoclave.
Next, the aseptic bottle 1 is set in place, and each piping is filled with aseptic water 2, and the pump 3, the three-way valve 21, and the syringe pump 22 are operated until the aseptic water in the pipings is replaced several times.
Since the alcohol is mixed with the aseptic water 2 at an arbitrary rate, the remaining alcohol is displaced with the aseptic water 2.
Afterwards, as daily facility maintenance, the piping is cleaned by using a piping cleaning reagent, the remaining bacteria in the piping system L1 are killed, and the ATP residue is consumed by the reagent reaction, and the piping is cleaned.
The hollow fiber filter 6 is a filter for passing water through hollow fibers made of rayon or the like, and generally there are few commercial products resistant to alcohol, but it is not required to exchange in the case of the filter resistant to alcohol. In the case of the hollow fiber filter 6 not resistant to alcohol, the measuring apparatus is stored in a state having a remaining alcohol in the inside by alcohol displacement, and when resuming the operation of the measuring apparatus, the parts are replaced with new ones, and the contamination risk in the piping is avoided, and the operation can be started again safely.

### [Storing method when shutting down the measuring apparatus of airborne floating bacteria for a short period]

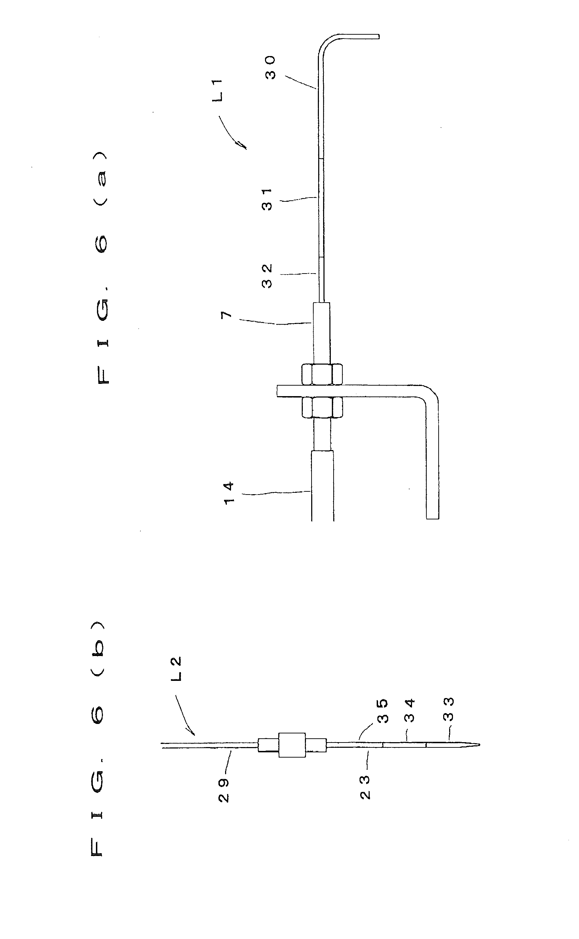

When shutting down the operation of the plant for a short period for a week or ten days or so during summer vacation or New Year holidays, a simple method may be employed in the method of replacing with alcohol in the piping.

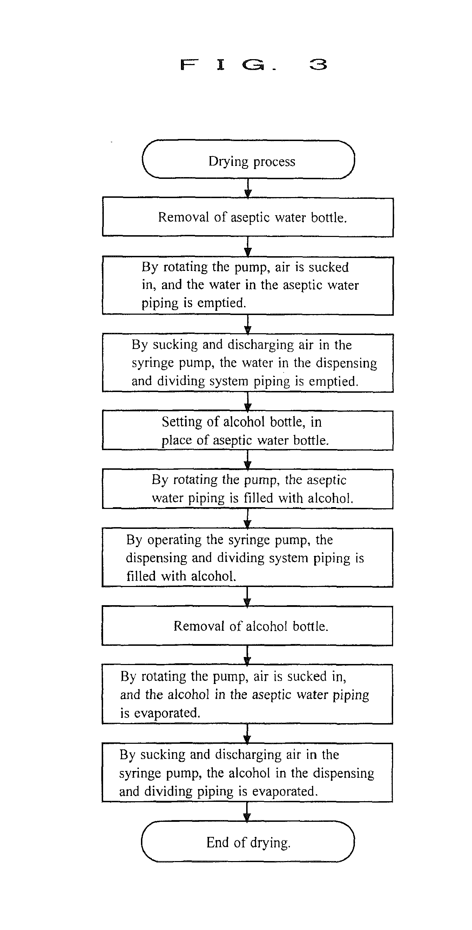

Fig. 3 shows an outline flow of the storing method when shutting down the measuring apparatus of airborne floating bacteria for a short period.
What differs largely in the procedure lies in the process of displacing with alcohol from the aseptic water in the nozzle leading ends, and storing directly in this state.
More specifically, the piping of the warm water system L1 formed as shown in Fig. 6 (a), in which the piping is filled with aseptic water in a same state as in an ordinary operation, an alcohol layer 30, an air layer 31, and a filled layer 32 of aseptic water are formed at the leading end part of the warm water nozzle 7 sequentially from the leading end part, and the pump 3 is operated reversely, and air and alcohol are sucked in from the leading end of the warm water nozzle 7.
On the other hand, the dispensing and diving piping L2 is as shown in Fig. 6 (b), in which the piping is filled with aseptic water in a same state as in an ordinary operation, an alcohol layer 33, an air layer 34, and a filled layer 35 of aseptic water are formed at the leading end part of the warm water nozzle 7 sequentially from the leading end part, and air and alcohol are sucked in from the dispensing and dividing nozzle 23.
In this case, the aseptic water bottle 1 at the other end of the piping system L is exchanged with an alcohol bottle, and the leading ends of the piping 11 and the piping 28 are immersed in alcohol.
In this manner, by shielding the inside of the piping of the piping system L with an alcohol layer, if the leading ends of the warm water nozzle 7 or the dispensing and dividing nozzle 23 with bacteria, diffusion of contamination into the inside can be prevented by the alcohol layer and the air layer.
On the other hand, the aseptic bottle 1 side at the other end is also shielded by alcohol, and contamination will not be diffused.

The operation flow when resuming the operation of the measuring apparatus after shutdown for a short period may conform to the operation flow after shutdown for along or intermediate period shown in Fig. 4.
In the case of shutdown for a short period, replacement of the hollow fiber filter 6 is not required.
The alcohol adhered to the leading end parts of the warm water nozzle 7 or the dispensing and dividing nozzle 23 can be completely evaporated and sterilized at the time of autoclave operation.
The pipings 11, 28 at the aseptic water bottle 1 side include regions of mixture of alcohol and aseptic water, but these regions may be displaced with aseptic water by passing aseptic water into the piping by several times of volume of the piping capacity.

### [When using sulfate buffer in place of aseptic water]

As explained so far in the embodiment, the procedure of filling the piping with aseptic water is explained, but the procedure is different when using sulfate buffer in place of aseptic water.
In the operation before shutdown for a long or intermediate period, while the sulfate buffer is being displaced with alcohol, by adding one process of displacement process of displacement from aseptic water to alcohol, the operation can be carried out without causing precipitates of sulfate buffer in the piping.

In the embodiment, it is explained about an example of single warm water system L 1 and dispensing and dividing system L2, but in measurement of airborne floating bacteria enhanced in the treating capacity by providing with plural warm water systems and plural dispensing and dividing systems, the contamination risk of bacteria when storing the measuring apparatus can be reduced by applying the procedure of the embodiment.

So far is explained about the embodiments of the storing method of the measuring apparatus of airborne floating bacteria of the invention, but the present invention is not limited to the above-described embodiments alone, but it is possible to change and modify freely so far as not to depart from the true spirit thereof.

### [Industrial Applicability]

The storing method of the measuring apparatus of airborne floating bacteria of the invention is capable of preventing contamination of the measuring apparatus with airborne floating bacteria during storage period of the measuring apparatus of airborne floating bacteria, and growth of airborne floating bacteria within the measuring apparatus, and hence it can be preferably applied when storing the measuring apparatus of airborne floating bacteria in the case of shutdown for a long or intermediate period or for a short period.
The above embodiments of the invention as well as the appended claims and figures show multiple characterizing features of the invention in specific combinations. The skilled person will easily be able to consider further combinations or sub-combinations of these features in order to adapt the invention as defined in the in the claims to his specific needs.

### [Description of the Reference Numerals]

- L: Piping system
- L1: Warm water system
- L2: Dispensing and dividing system
- 1: Aseptic water bottle
- 2: Aseptic water
- 3: Pump
- 4: Piping connector
- 5: Warm water heater
- 6: Hollow fiber filter
- 7: Warm water nozzle
- 8: Capturing cartridge
- 9: Dissolving heater
- 10: Piping
- 11: Piping
- 12: Peristaltic tube
- 13: Fixed tube
- 14: Movable part piping
- 21: Three-way valve
- 22: Syringe pump
- 23: Dispensing and dividing nozzle
- 24: Tube
- 25: Light emission measurement tube
- 26: Darkroom
- 27: Photomultiplier
- 28: Piping
- 29: Piping
- 30: Alcohol layer (warm water nozzle)
- 31: Air layer (warm water nozzle)
- 32: Aseptic water filled layer (warm water nozzle)
- 33: Alcohol layer (dispensing and dividing nozzle)
- 34: Air layer (dispensing and dividing nozzle)
- 35: Aseptic water filled layer (dispensing and dividing nozzle)

## Claims

1. A storing method of a measuring apparatus of airborne floating bacteria, being a storing method of a measuring apparatus of airborne floating bacteria, **characterized by** capturing airborne floating bacteria on a gel of a capturing carrier, forming into a sol in the measuring apparatus, and measuring the light emission of the ATP, wherein the aseptic water existing in the piping system of the measuring apparatus is displaced with alcohol, and dried, and the measuring apparatus of airborne floating bacteria is stored.

2. The storing method of the measuring apparatus of airborne floating bacteria according to claim 1 of the present invention, wherein the hollow fiber filter is replaced when resuming operation of the measuring apparatus of airborne floating bacteria.

3. A storing method of a measuring apparatus of airborne floating bacteria, being a storing method of a measuring apparatus of airborne floating bacteria, **characterized by** capturing airborne floating bacteria on a gel of a capturing carrier, forming into a sol in the measuring apparatus, and measuring the light emission of the ATP, wherein the nozzle section of the piping system of the measuring apparatus is filled with alcohol, and the measuring apparatus of airborne floating bacteria is stored in this state.

4. The storing method of the measuring apparatus of airborne floating bacteria according to claim 3 of the present invention, wherein the operation of the measuring apparatus of airborne floating bacteria is resumed by evaporating the alcohol packed in the nozzle section of the piping system of the measuring apparatus at the time of autoclave sterilization.
